(19)

Europäisches
Patentamt
European
Patent Office
Office européen
des brevets

(11)　　**EP 1 985 238 A2**

(12)　　**EUROPEAN PATENT APPLICATION**

(43) Date of publication:
　　**29.10.2008　Bulletin 2008/44**

(51) Int Cl.:
　　**A61B 8/06** (2006.01)　　　**G01S 15/89** (2006.01)

(21) Application number: **08155039.4**

(22) Date of filing: **23.04.2008**

(84) Designated Contracting States:
　　**AT BE BG CH CY CZ DE DK EE ES FI FR GB GR
　　HR HU IE IS IT LI LT LU LV MC MT NL NO PL PT
　　RO SE SI SK TR**
　　Designated Extension States:
　　**AL BA MK RS**

(30) Priority: **24.04.2007　US 926124 P
　　　　　　21.04.2008　US**

(71) Applicants:
　　• **Siemens Medical Solutions USA, Inc.
　　　Malvern, PA 19355 (US)**

• **Amid SRL
　00191 Roma (IT)**

(72) Inventors:
　　• **Tonti, Giovanni
　　　67039, Sulmona (IT)**
　　• **Pedrizzetti, Gianni
　　　59100, Prata (IT)**

(74) Representative: **Hazzard, Alan David
　　Siemens Aktiengesellschaft
　　Postfach 22 16 34
　　80506 Munich (DE)**

(54)　　**Flow characteristic imaging in medical diagnostic ultrasound**

(57)　　Flow is characterized in medical diagnostic ultrasound imaging. The flow information over time for each spatial location may be broken down (36) into component parts using Fourier analysis. For example, the average or steady state component of the flow at each location may be determined (37) and used for imaging (40). The first harmonic may likewise be used. Imaging using one or more component parts of the flow information over time may provide diagnostically useful information. In addition or independently, the flow information may be analyzed to identify (42) a vortex. The vortex characteristics may provide diagnostically useful information.

Figure 1

EP 1 985 238 A2

**Description**

RELATED APPLICATIONS

The present patent document claims the benefit of the filing date under 35 U.S.C. §119(e) of Provisional U.S. Patent Application Serial No. 60/926,124, filed April 24, 2007, which is hereby incorporated by reference.

BACKGROUND

The present embodiments relate to ultrasound imaging of flow. Flow is characterized using medical diagnostic ultrasound.

Blood in the heart and in the circulatory system is expected to flow in a physiological appropriate manner. The flow pattern of blood may respond quickly to change due to pathology or stress. For example, vortices are produced downstream of a stenosis, or stagnating intraventricular circulation is found next to a hypokinetic, ischemic wall. The ability to evaluate the blood flow may improve diagnosis and therapeutic processes. Non-invasive evaluation of the blood flow may be beneficial.

Blood flow may be non-invasively analyzed using magnetic resonance imaging (MRI). MRI requires large equipment and is not of practical use in clinical routine. In addition, flow calculation requires an acquisition at a high frame rate that cannot normally be achieved in MRI.

Ultrasound imaging provides blood flow information non-invasively. Doppler imaging indicates the velocity of blood flow. For a given range gate (spatial location), a time profile or spectrum of velocity is provide by spectral, pulsed wave, or continuous wave Doppler imaging. For spatial distribution of the velocity, color or flow Doppler indicates velocities over a two or three-dimensional region. Doppler echocardiography is widely used in clinical practice and represents a valuable diagnostic tool. However, Doppler echography measures the velocity along the direction of a scan line, detecting the velocity at which blood is moving towards or away from the transducer. Motion in other directions is not so readily detectable even though the blood may move in any direction. Multiple transducers, varied scanning techniques, correction based on a determined flow direction, or other approaches may be used to determine an actual velocity, but may have limited practical use.

Flow can be analyzed by looking at a time sequence of images, such as B-mode and Doppler images. Often one or few special instants are considered to reduce the large amount of information available. The diagnostic process is a synthesis of information, where such synthesis of information should be based on a large amount of information available in order to increase the reproducibility of the result. Considering only a subset of the information may limit diagnosis. Considering the entire time sequence may be time consuming or difficult.

BRIEF SUMMARY

By way of introduction, the preferred embodiments described below include methods, instructions, and systems for characterizing flow in medical diagnostic ultrasound imaging. The flow information over time for each spatial location may be broken down into component parts using Fourier analysis. For example, the average or steady state component of the flow at each location may be determined and used for imaging. The first harmonic may likewise be used. Imaging using one or more component parts of the flow information over time may provide diagnostically useful information. In addition or independently, the flow information may be analyzed to identify a vortex. The vortex characteristics may provide diagnostically useful information.

In a first aspect, a method is provided for characterizing flow in medical diagnostic ultrasound imaging. Flow values are detected for each of a plurality of cardiac locations over at least a portion of a heart cycle. The flow values for each of the cardiac locations are time-decomposited into Fourier components. An image representing the cardiac locations is generated as a function of at least one of the Fourier components.

In a second aspect, a computer readable storage medium has stored therein data representing instructions executable by a programmed processor for characterizing flow in medical diagnostic ultrasound imaging. The storage medium includes instructions for determining flow for a plurality of locations in a heart over a period, calculating an average flow field for the period, and displaying the average flow field.

In a third aspect, a method is provided for characterizing flow in medical diagnostic ultrasound imaging. Flow values for each of a plurality of cardiac locations are detected with ultrasound. Vortex information is calculated as a function of the flow values. The vortex information corresponding to a vortex associated with a plurality of the cardiac locations.

In a fourth aspect, a computer readable storage medium has stored therein data representing instructions executable by a programmed processor for characterizing flow in medical diagnostic ultrasound imaging. The storage medium includes instructions for determining flow, for a plurality of locations in a heart, from ultrasound information, extracting at least one characteristic of a vortex from the flow, and outputting the at least one characteristic of the vortex.

The present invention is defined by the following claims, and nothing in this section should be taken as a limitation on those claims. Further aspects and advantages of the invention are discussed below in conjunction with the preferred embodiments and may be later claimed independently or in combination.

BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.

Figure 1 is a flow chart diagram of one embodiment of a method for characterizing flow in medical diagnostic ultrasound imaging;

Figure 2 shows example ultrasound images of the left ventricle at different times during filling; and

Figure 3 shows one embodiment of the ultrasound imaging of Figure 2 with velocity trajectories mapped over the images;

Figure 4 shows one embodiment of a flow pattern image; and

Figure 5 is a block diagram of one embodiment of a system for characterizing flow in medical diagnostic ultrasound imaging.

DETAILED DESCRIPTION OF THE DRAWINGS AND PRESENTLY PREFERRED EMBODIMENTS

The flow pattern may be evaluated using fluid dynamics applied to ultrasound information. A two or three-dimensional velocity vector field is determined from B-mode, contrast agent, or other ultrasound imaging. For practical usability for diagnostic and therapeutic purposes, the flow is visualized using time-decomposition or Fourier components. Quantitative results provide synthesis in physically-based terms. Ultrasound imaging systems or offline analysis of ultrasound imaging may provide additional diagnostic support by outputting images based on the time-decomposition and/or identified vortex and flow information.

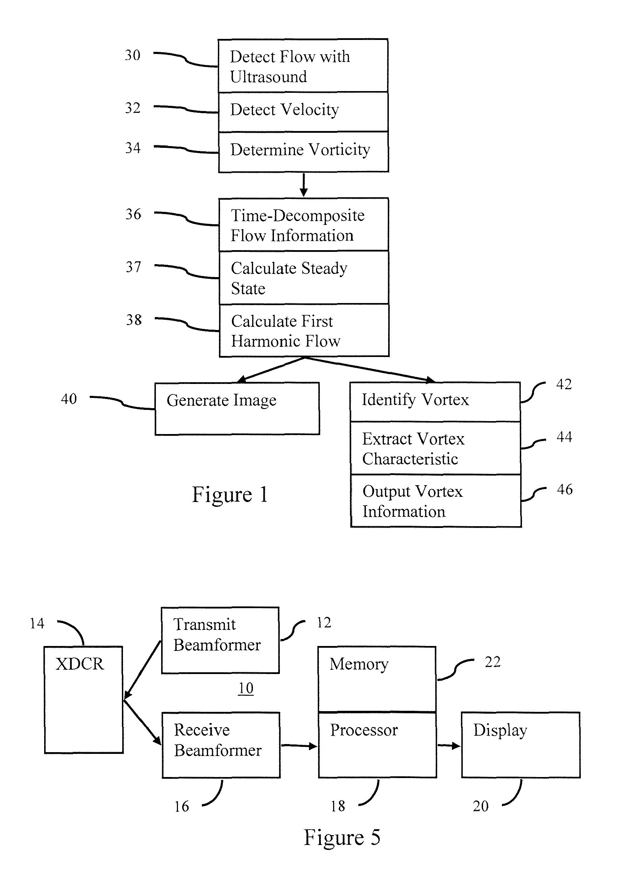

Figure 1 shows a method for characterizing flow in medical diagnostic ultrasound imaging. The method is implemented by the system of Figure 5 or a different system. Additional, different, or fewer acts may be provided. For example, acts 34, 37, and/or 38 are not provided. For example in determining vortex information, acts 34, 36-38 and 40 are not provided. For example in generating an image, acts 34, 37, 38, 42, 44, and/or 46 are not provided. The acts are performed in the order described or shown, but may be performed in other orders.

In act 30, flow is determined for a plurality of locations. Flow values are detected for each of the plurality of locations. The locations are for full or sparse sampling of a two or three-dimensional region. For example, flow is determined for one or more two-dimensional scan regions or a region of interest, such as associated with a color or Doppler box.

The flow values are determined for a fluid region. A region of the circulatory system may be scanned. Flow in the cardiac chambers or circulatory tree, is detected. Alternatively, flow in other fluid regions, such as the intestinal or urinary system, may be detected.

The flow is determined over a period. Different flow values for a given spatial location are determined for different times. For example, flow is determined over at least a portion of a heart cycle, such as detecting the flow values for at least an entire heart cycle. Flow values over multiple cycles may be combined or averaged, such as averaging flow values representing a same time relative to the repeating heart cycle. In one embodiment, flow values from the most recently acquired three heart cycles are combined to provide a representative heart cycle. The flow during an entire or portion of a cycle may be sampled at any desired frequency, such as at two or more instants during one heartbeat.

In act 32, the flow is detected by detecting velocity. Velocity may be detected using Doppler techniques. A plurality of pulses is fired along a scan line. The change in frequency of the echoes indicates a velocity of objects, such as blood, along the scan line. Angle correction is provided, such as the user inputting different trajectories. Correlation functions may be used to determine the velocity. For example, a plurality of B-mode scans is performed. Different regions are tracked using speckle, contrast agents, or features to determine offset or change in position. The velocity is determined based on the change in position and the time between scans. Other now known or later developed ultrasound technique for determining velocity may be used.

In one embodiment, the velocity is detected as a function of intensity returns from contrast agents. Any contrast agent imaging technique may be used, such as B-mode imaging, harmonic response imaging, loss of correlation imaging, cubic fundamental imaging, or Doppler imaging.

The movement of contrast agents is tracked to determine velocity. For example, the flow calculation is based on the analysis of a sequence of, at least two, grey scale images. Figure 1 shows two B-mode images of tissue and contrast agents. The ultrasound images are of the left ventricle at two consecutive instants during filling by the contrast agents. A small infusion of contrast agent may improve visualization of flowing fluids. The endocardial border is shown as a dotted line, and the arrow indicates the direction of blood flow. The border may be determined automatically, manually, or not determined. The direction of blood flow is shown to assist in understanding, but may be provided for imaging. The left image shows the brighter flow jet entering the cavity. A starting vortex is generally visible on the right side of the jet head. The right image is acquired after approximately 80 ms. The fluid has entered further into the cavity as shown by the contrast agent return. The flow velocity is the distance traveled by the fluid between the two images divided by the time interval.

Blood, when subjected to ultrasound waves, produces a backscatter of the ultrasound signal. Backscatter from a fixed element (e.g., stagnating blood) is essentially equal in two consecutive images. Backscatter from a moving element (e.g., flowing blood cells or contrast agent) is found displaced from one frame to the following. An analysis of backscatter permits evaluation of the displacement of the element from one frame to the next. This type of processing has the same theoretical basis as Particle Image Velocimetry (PIV) method developed in experimental fluid mechanics. Instead of light backscatter, the PIV is applied to ultrasound backscatter from contrast agents. The brightness of returns from the contrast agents is conserved. The movement of the contrast agent bolus in two spatial regions, each one in two consecutive frames, is tracked to determine the velocity at the different locations. A total derivative of brightness in time, where total derivative means the derivative following the local motion, is zero when the agents undergoes a rigid motion. Such a total derivative is mathematically evaluated by the time derivative taken between two corresponding regions, plus the multiplication of the expected displacement between such regions with the spatial gradient. In real acquisitions, the spatial displacements that minimizes such total derivative is estimated by minimizing the least squares fit of the total derivative in the examination region. Displacement may be determined from the brightness information provided by the ultrasound return.

Different flow detections may be combined. For example, PIV of contrast agent return generates one set of flow values for a region. Doppler velocity and/or speckle correlation flow values may be averaged with the PIV flow values or used to confirm accuracy. Even with limited accuracy or undesired variance, the estimated velocity vectors may be used to evaluate the flow pattern, such as the arrangement of vortices or extension of strain regions.

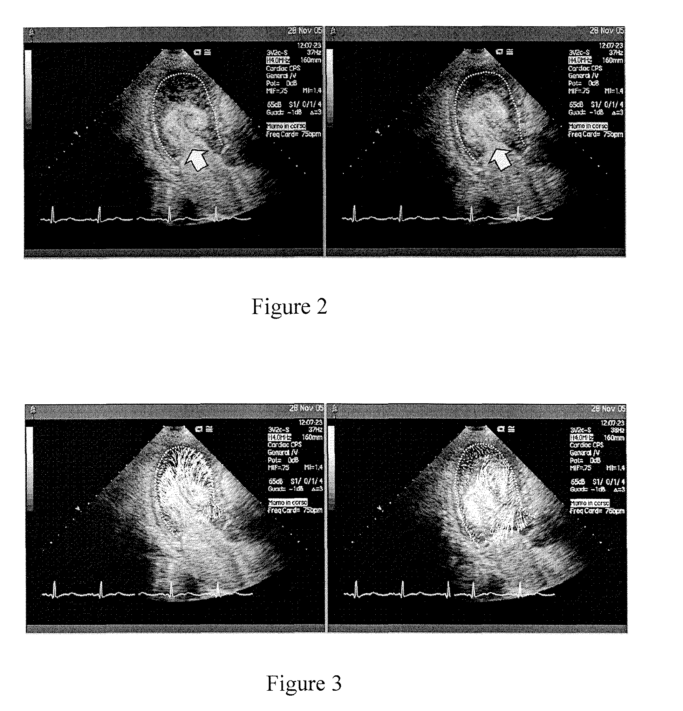

The velocity vector at points in the analyzed spatial domain is provided for a given instant in time or for a frame. The velocity vectors at different times for the points are also determined. Blood flow in a cardiovascular region is described by the value of a vector v(x, t) where x is a vector of spatial coordinates that describe all points in two or three-dimensional space and t is the time. In two-dimensional imaging, the velocity is a two-dimensional vector, typically described by its x and y components, $v_x(x,y,t)$ and $v_y(x,y,t)$ that vary along the x and y directions. Figure 3 shows the velocity field inside the left ventricle at two instants during one heartbeat. The computed velocity vectors are superimposed over the B-mode images of Figure 2. The arrows represent the computed velocity vectors or trajectories. Velocity vectors computed for a sparse sampling, irregular or points with higher variance are overlaid on the image. Each arrow starts at the point for which the vector is calculated to enhance visualization of blood motion. The left image corresponds to one instant during ventricular filling (left image in Figure 2) when a flow jet is entering the cavity. The right image is at a later instant, diastases, when the intraventricular is well visible. The number of arrows may be increased or decreased.

Other flow values may be determined. In act 34, the vorticity is determined for at least some of the locations for which velocity was determined. The vorticity of the velocity is determined. The vorticity is the curl of the velocity vector and is calculated from the velocity vectors. The vorticity is computed from a curl combination of derivatives of the velocity field: in two dimensional flow the vorticity is a scalar quantity that is given by the difference between the x-derivative of the $v_y(x,y,t)$ and the y-derivative of the $v_x(x,y,t)$; in three-dimensional fields the vorticity is also a vector by similar differences between velocity derivatives. The vorticity represents the skeleton of the flow field. Other approaches for determining vorticity may be used. The velocity, vorticity, other flow value, or combinations thereof are used for imaging and/or determining vortex information.

In act 36, the flow values for each of the cardiac locations are time-decomposed into Fourier components. The flow values are velocity, vorticity, or other flow values. Two or more flow values representing a same location are used as a series in the temporal domain for conversion into the frequency domain. The time-decomposition is repeated for each desired spatial location.

Time-decomposition may maintain global features in a simpler form for imaging or determining a flow characteristic. Since cardiovascular flow is time-periodic, time decomposition of the flow into Fourier harmonics is provided. For example, the velocity or the vorticity ω(x,t) at each point with coordinates x is a periodic function of time that can be expressed in Fourier series as:

$$\omega(x,t) = \omega_0(x) + \omega_1(x)\cos\left(\tfrac{2\pi}{T}t + \varphi_1(x)\right) + \sum_{k=2,N}\omega_k(x)\cos\left(\tfrac{2k\pi}{T}t + \varphi_k(x)\right)$$

where $\omega_0(x)$ is the average flow field of one heartbeat, $\omega_1(x)$ is the fundamental (first) harmonic (sinusoidal) that represents the main pulsatile contribution to the flow, and $\varphi_1(x)$ is the corresponding phase that is a measure of the synchronicity of the pulsatile contribution. The further terms with subscript k are the higher harmonics that correspond to the additional unsteady Fourier components.

In act 37, the average flow field is calculated. The average is over any time, such as over a heart cycle. For a plurality of spatial locations, the average, or other steady state value (median) is calculated. For example, the DC component of the Fourier series for the flow over the period is calculated. The steady state Fourier component indicates a steady state value or the "steady-streaming" flow. In another example, the flow values are averaged without Fourier analysis. A large amount of information about the overall structure of the periodic flow field may be represented by the average flow field. The steady state indicates statically the basic signature of flow dynamics over the entire heartbeat or other period. The steady-streaming field is a fundamental synthesis of the flow and may provide a statistically reproducible result.

In act 38, a first harmonic flow field is calculated. The first pulsatile harmonic, $\omega_1(x)$, of the Fourier series represents, hierarchically, another flow field. The first harmonic synthesizes the information over the heartbeat or other period. The first harmonic corresponds to the fundamental pulsatile contribution. For static information, the magnitude of the first harmonic component of the Fourier series is calculated for each spatial location. The phase $\varphi_1(x)$ may also provide pulsatility information. The magnitude and/or phase for the first harmonic may be used to indicate flow characteristics of the region of interest over a period.

Other techniques to represent the unsteady or pulsatile contributions in the flow field may be used. Such unsteadiness measures include, but are not limited to, the deviation from the average, the variance within the heartbeat, the sum of absolute values, or the sum of squares that is also related to the kinetic energy when applied to velocity or entropy when applied to vorticity.

In act 40, an image is generated. The image represents ultrasound information from the scanned region, such as cardiac locations. Color, grayscale, or both may be used. The image is generated by setting red, green, and blue values (RGB), but YUV or other display formats may be used. The image values are set as a function of one or more input values. Any modulation or mapping function may be used.

The image is a two-dimensional image. In alternative embodiments, the image is rendered as a representation of a three-dimensional volume. Any rendering may be used, such as surface rendering, projection, or other techniques.

The image may show the flow values, such as setting the pixels as a function of velocity and/or vorticity. For example, a gray scale tissue or B-mode image may be overlaid with a color velocity or vorticity image. The image may include velocity or vorticity information from tracking the contrast agents and/or from Doppler detection. Any now known or later developed Doppler, B-mode, and/or contrast agent imaging may be used.

In one embodiment, the image values are set as a function of one or more of the Fourier components. The intensity, color, hue, brightness, combinations thereof, or other characteristic of the image are set based on one or more Fourier components. One characteristic may be set as a function of one component and another characteristic may be set as a function of another component. Different characteristics may be set based on different input values. One or more characteristic may be set based on a combination of two input values, such as setting color using a look-up table based on inputting the first harmonic magnitude and the phase.

For example, the image displays the average flow field. The image is generated as a function of the steady state Fourier component or average flow velocity. The "steady-streaming" field $\omega_0(x)$ does not depend on time and may be visualized as a simple image. The steady streaming flow imaging (average flow, zeroth harmonics of the flow time-Fourier decomposition) provides a synthesis of the time periodic cardiovascular flow to evidence the flow structure for diagnostic purposes. The arrangement of the dominant vortices may be visible in the image. The dominant vortices represent the fundamental quantity in flow-based diagnostics.

In another example, the image is generated as a function of the first harmonic or another measure of unsteadiness. The image represents pulsatility over the period. The first and higher harmonics contain fundamental information about the pulsatility of the vortex structures. The image is static, but multiple images may be created for sequential periods with or without overlap. In one embodiment, the phase is mapped to a binary color coding, such as red for before a time in the heart cycle and blue for after a time in the heart cycle. The hue and/or brightness are more gradually mapped.

Other features may be added to the image. For example, features indicating trajectory, streamlines, streaklines, gradients, kinetic energy, other fluid dynamics features, or combinations thereof. The features are graphic overlays, such as points, lines, arrows, spirals, grids, or other shapes. Alternatively, the features are used as an input for determining color, hue, or brightness. Velocity is a multidimensional, time-varying, vector field that may not be immediately assessed by visualization. The features of the flow can often be better visualized by assessing some derived quantities that are directly related with the effective flow dynamics. Streamlines, trajectories, and streaklines are integral quantities or quantities that require integration of the flow values along time and/or space. Streamlines show the path of flow where the streamlines are everywhere tangent to the flow vector, such as the velocity vector. Trajectories indicate a history of the path, such as by a vector indicating the direction and magnitude of flow from a particular point. Kinetic energy and flow gradients are local or differential quantities, such as quantities based on the flow values or a differential of flow along time and/or space.

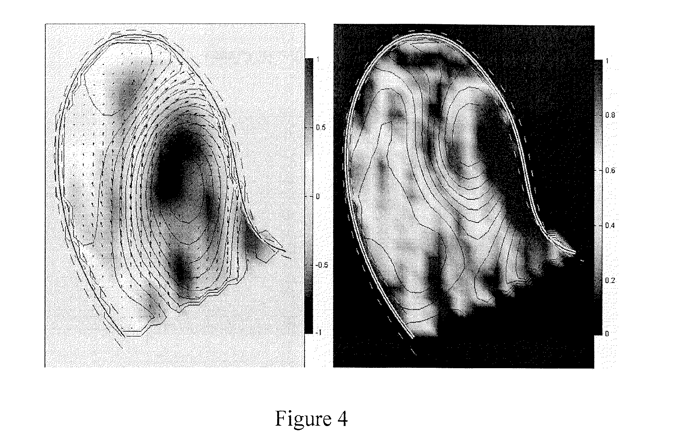

Figure 4 shows example embodiments of two images to be displayed separately or together. The image on the left shows the steady stream of the flow in the left ventricle. The average or steady streaming vorticity is color mapped with shades of blue representing counter clockwise vorticity and shades of red representing clockwise vorticity. Arrows at regular, sparse locations are superimposed to represent the steady streaming velocity vectors (trajectories). Lines in the flow field are streamlines of the steady streaming velocity. The fundamental left ventricular vortex is well visible as indicated by the streamlines, trajectories, and color.

The image on the right shows the fundamental harmonic of pulsatile flow. The first harmonic magnitude of the vorticity field is color mapped with blue representing little or no pulsitivity and red representing maximum pulsitivity. The shades in between provide a graduation of the magnitude of the first harmonic. Streamlines are overlaid. The streamlines are of the first harmonic of the velocities.

Static images representing the steady state and unsteady features (pulsatile) components over a period, such as a heart cycle are provided. These images represent a synthesis of information. Both images in Figure 4 also include lines representing the endocardial border at systolic, mean, and diastolic positions. Different, fewer, or additional features may be provided. Only vorticity, only velocity, or other combinations of velocity and vorticity flow values may be used for either of the images. Different color scales or mapping may be provided. Different combinations or only one of the two images may be used. The images may be updated regularly, such as generating the image based on a moving window of one or more heartbeats. As data for another heartbeat is acquired, the data from a previously acquired heartbeat is replaced for generating the image.

The image is displayed in two dimensions. The velocity field may be determined from a two-dimensional scan. Such a velocity field does not include a detected out-of-plane velocity. For a three-dimensional scan, the velocity has a three-dimensional vector. The flow exiting/entering the image plane is evaluated from the velocity divergence from the image. The velocity divergence is the sum of in-line derivatives of the flow: the sum of the x-derivative of $v_x(x,y,t)$ with the y-derivative of $v_y(x,y,t)$. This out-of plane velocity information may be reduced for generating the image or a feature. For example, the velocity streamlines and trajectories may be generated from velocity information remaining after reducing or removing out-of plane components of the velocity vector. The steady streaming in-plane streamlines correspond to streamlines along the divergence-free velocity field where the cross-flow, out-of-plane component of velocity is reduced. The divergence is removed by spatial derivatives by subtracting from the velocity field a potential flow corresponding to exactly the same divergence, that may be computed by application of the inverse Laplacian operator to the divergence. The vorticity or other fluid dynamical quantities may be determined from the divergence free velocity. In alternative embodiments, divergence is not removed from the velocity field.

In act 42, a vortex is identified. In one embodiment, the vortex is identified from the average flow field. In other embodiments, the vortex is identified from instantaneous flow values at physiologically relevant instants, other Fourier components, and/or features derived from the flow values or Fourier components.

The vortex is associated with a plurality of cardiac locations. The vortex is a region of compact vorticity. Adjacent vorticity values having higher magnitude may indicate a vortex. In one embodiment, the vorticity is calculated as the derivative of the velocity. In another embodiment, a smoother version of vorticity, the stream function of the flow, is evaluated by application of the inverse Laplacian operator to the vorticity. In another embodiment, a different measure of vortical flow may be used, like pressure, the Laplacian of pressure, an eigenvalue of the velocity gradient tensor or of its combination. The vortical measure values may be low pass filtered to spatially smooth the values or remove high frequency variance. The local maximums of vorticity or another vortical measure are identified. For example, the highest one, two, or other numbers of local maximum values are located. Any desired separation between local maximums may be required, such as maximum values separated by tissue and/or flow below a threshold level. As another example, a region of relatively high vorticity is determined as a function of a vorticity density. The region with a highest vorticity density is identified by locating the position of vorticity maximums, locating the compact regions around such maximums where vorticity value is above a certain threshold, and extracting the region where the sum of vorticity inside such regions is largest. Other processing may be used, such as clustering or region growing, to identify a vortex.

Other flow structure may be identified. For example, shear layers or boundary layers are identified from the vorticity values. A shear layer is an elongated layer of friction between streams with differential motion. A boundary layer is a shear layer next to a wall. Other diagnostic indicators of flow may be extracted.

In act 44, a characteristic of a vortex is extracted. The location, size, shape, strength, or combinations thereof are determined for one or more vortices. The characteristics are extracted from the flow. Instantaneous flow values or the Fourier components of the flow values are analyzed. For example, the time evolution of the vortex information is determined from the analysis of flow field at all the available times. Global information is determined from further analysis of such time-varying vortex information or from analysis of the at least one Fourier component, such as calculating from the steady state Fourier component.

The location is determined from the point of maximum steady state vorticity. Alternatively, the location is determined as a center of an area or volume defined by the size. Other approaches may be used.

The size is determined by a threshold. A contour or location along radii where the vorticity values are a threshold amount down from the magnitude defines a circumference. Region growing, clustering, or other algorithms may be used. For example, the streamlines surrounding the maximum vorticity are used as the circumference of the vortex. The area, volume, length, width, height, other measure, or combinations thereof indicate the size.

The strength is an average vorticity in the vortex, such as the vortex as defined by the size calculation. Other measures of strength may be used, such as the magnitude of the maximum, its circulation, the integral of vorticity, another measure of vortical motion, a size weighted average, a number of spatial locations included in the vortex, or a characteristic of the variance or decay within the vortex.

In act 46, the characteristic of the vortex is output. The output is to memory or displayed to the user. For example, one or more characteristics of each vortex are output as text on, over, or adjacent to the image generated in act 40. The vortex may be highlighted or a vortex indication overlaid on the image based on the characteristics. For example, different color mapping, shading, or a graphic highlight the vortex relative to other locations in the image is provided.

Figure 5 shows one embodiment of a system 10 for characterizing flow in medical diagnostic ultrasound imaging. The system 10 implements the method of Figure 1 or other methods. The system 10 includes a transmit beamformer 12, a transducer 14, a receive beamformer 16, an image processor 18, a display 20, and a memory 22. Additional, different or fewer components may be provided. For example, a user input is provided for manual or assisted selection of display maps, vortex properties to be determined, region of interest selection, border definition, or other control. The system 10 is a medical diagnostic ultrasound imaging system. In alternative embodiments, the system 10 is a personal computer, workstation, PACS station, or other arrangement at a same location or distributed over a network for real-time or post acquisition imaging, so may not include the beamformers 12, 16 and transducer 14.

The transmit beamformer 12 is an ultrasound transmitter, memory, pulser, analog circuit, digital circuit, or combinations thereof. The transmit beamformer 12 is operable to generate waveforms for a plurality of channels with different or relative amplitudes, delays, and/or phasing. Upon transmission of acoustic waves from the transducer 14 in response to the generated waves, one or more beams are formed. A sequence of transmit beams are generated to scan a two or three-dimensional region. Sector, Vector®, linear, or other scan formats may be used. The same region is scanned multiple times. For flow or Doppler imaging, a sequence of scans is used. In Doppler imaging, the sequence may include multiple beams along a same scan line before scanning an adjacent scan line. For flow imaging to track movement of speckle or contrast agents, scan or frame interleaving may be used (i.e., scan the entire region before scanning again). In alternative embodiments, the transmit beamformer 12 generates a plane wave or diverging wave for more rapid scanning.

The transducer 14 is a 1-, 1.25-, 1.5-, 1.75- or 2-dimensional array of piezoelectric or capacitive membrane elements. The transducer 14 includes a plurality of elements for transducing between acoustic and electrical energies. The transducer 14 may be handheld, such as for positioning on a patients skin near an acoustic window. In other embodiments, the transducer 14 is sized and shaped for use inside a patient, such as on a transesphogeal probe or a catheter for imaging the heart. Receive signals are generated in response to ultrasound energy (echoes) impinging on the elements of the transducer. The elements connect with channels of the transmit and receive beamformers 12, 16.

The receive beamformer 16 includes a plurality of channels with amplifiers, delays, and/or phase rotators, and one or more summers. Each channel connects with one or more transducer elements. The receive beamformer 16 applies relative delays, phases, and/or apodization to form one or more receive beams in response to each transmission. In alternative embodiments, the receive beamformer 16 is a processor for generating samples using Fourier or other transforms.

The receive beamformer 16 may include a filter, such as a filter for isolating information at a second harmonic or other frequency band relative to the transmit frequency band. Such information may more likely include desired tissue, contrast agent, and/or flow information. In another embodiment, the receive beamformer 16 includes a memory or buffer and a filter or adder. Two or more receive beams are combined to isolate information at a desired frequency band, such as a second harmonic, cubic fundamental or other band.

The receive beamformer 16 outputs beam summed data representing spatial locations. Data for a single location, locations along a line, locations for an area, or locations for a volume are output. Dynamic focusing may be provided. The data may be for different purposes. For example, different scans are performed for B-mode or tissue data than for contrast agent data.

The image processor 18 is a B-mode detector, Doppler detector, pulsed wave Doppler detector, correlation processor, Fourier transform processor, application specific integrated circuit, general processor, control processor, field programmable gate array, digital signal processor, analog circuit, digital circuit, combinations thereof or other now known or later developed device for detecting and processing information for display from beamformed ultrasound samples.

In one embodiment, the image processor 18 includes one or more detectors and a separate processor. The processor is a control processor, general processor, digital signal processor, application specific integrated circuit, field programmable gate array, network, server, group of processors, data path, combinations thereof or other now known or later developed device for determining flow values from 2D or volumetric data, performing Fourier transforms, generating an image, and calculating vortex properties. For example, the processor 18 performs any combination of one or more of the acts shown in Figure 1.

The image processor 18 operates pursuant to instructions stored in the memory 22 or another memory. The processor 18 is programmed for characterizing flow in medical diagnostic ultrasound imaging. The memory 22 is a computer readable storage media. The instructions for implementing the processes, methods and/or techniques discussed herein are provided on the computer-readable storage media or memories, such as a cache, buffer, RAM, removable media, hard drive or other computer readable storage media. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. Likewise, processing strategies may include multiprocessing, multitasking, parallel processing, and the like. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU or system.

The memory 22 may alternatively or additionally be used for storing data. Flow values, such as velocity and vorticity, image values, vortex characteristics, image overlays, display maps or look-up tables, or other data is stored. The stored data is used for processing, calculating, or generating.

The display 20 is a CRT, LCD, projector, plasma, or other display for displaying two-dimensional images or three-dimensional representations. The display 20 displays ultrasound images, such as velocity, vorticity, Fourier component, steady state, first harmonic, or other images. The display 20 may display representations of or quantities for a vortex.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the spirit and scope of this invention.

## Claims

1. A method for characterizing flow in medical diagnostic ultrasound imaging, the method comprising:

   detecting (30) flow values for each of a plurality of cardiac locations over at least a portion of a heart cycle;
   time-decompositing (36) the flow values for each of the cardiac locations into Fourier components; and
   generating (40) an image, representing the cardiac locations, as a function of at least one of the Fourier components.

2. The method of Claim 1 wherein the flow values for at least an entire heart cycle are detected (30), wherein the at least one of the Fourier components comprises a steady state Fourier component, and wherein generating (40) the image comprises generating (40) the image as a function of the steady state Fourier component.

3. The method of Claim 1 wherein the at least one of the Fourier components comprises a first harmonic, and wherein generating (40) the image comprises generating (40) the image as a function of the first harmonic.

4. The method of Claim 1 wherein detecting (30) flow values comprises detecting (32) velocity as a function of intensity returns from contrast agents.

5. The method of Claim 1 wherein detecting (30) flow values comprises determining (34) vorticity of velocity information.

6. The method of Claim 1 wherein generating (40) the image comprises color mapping as a function of the at least one of the Fourier components.

7. The method of Claim 6 wherein generating (40) the image further comprises indicating velocity trajectory, velocity streamlines, or combinations thereof.

8. The method of Claim 7 further comprising:

   reducing out-of plane velocity information, the velocity streamlines being a function of remaining velocity information;

   wherein generating (40) the image comprises generating (40) a two-dimensional image.

9. The method of Claim 1 further comprising:

   determining (44) vortex information as a function of the at least one Fourier component.

10. The method of Claim 9 wherein determining (44) vortex information comprises determining a location, size, strength, or combinations thereof of at least one vortex.

11. In a computer readable storage medium (22) having stored therein data representing instructions executable by a programmed processor (18) for characterizing flow in medical diagnostic ultrasound imaging, the storage medium (22) comprising instructions for:

   determining (30) flow for a plurality of locations in a heart over a period;
   calculating (37) an average flow field for the period; and
   displaying (40) the average flow field.

12. The computer readable storage medium (22) of Claim 11 wherein determining (30) flow comprises determining (36) vorticity for the locations.

13. The computer readable storage medium (22) of Claim 11 wherein calculating (37) the average flow field comprises determining a DC component of a Fourier series for the flow over the period.

14. The computer readable storage medium (22) of Claim 11 further comprising:

   identifying (42) a vortex in the average flow field; and
   determining (44) a characteristic of the vortex.

15. A method for characterizing flow in medical diagnostic ultrasound imaging, the method comprising:

   detecting (30), with ultrasound, flow values for each of a plurality of cardiac locations; and
   calculating (44) vortex information as a function of the flow values, the vortex information corresponding to a vortex associated with a plurality of the cardiac locations.

16. The method of Claim 15 wherein calculating (44) vortex information comprises determining a location, size, strength, or combinations thereof of the vortex.

17. The method of Claim 15 wherein the flow values for at least an entire heart cycle are detected, further comprising:

   time-decompositing (36) the flow values for each of the cardiac locations into Fourier components, including a steady state Fourier component; and
   generating (40) an image, representing the cardiac locations, as a function of the steady state Fourier component;

   wherein calculating (44) as a function of the flow values comprises calculating as a function of the steady state Fourier component.

18. The method of Claim 15 wherein detecting (30) flow values comprises determining (34) vorticity of velocity information, and wherein calculating (44) comprises identifying (42) a region of relatively high vorticity as a function of a vorticity density.

19. In a computer readable storage medium (22) having stored therein data representing instructions executable by a programmed processor (18) for characterizing flow in medical diagnostic ultrasound imaging, the storage medium (22) comprising instructions for:

determining (30) flow, for a plurality of locations in a heart, from ultrasound information;
extracting (44) at least one characteristic of a vortex from the flow; and
outputting (46) the at least one characteristic of the vortex.

20. The computer readable media of Claim 19 wherein extracting (44) the at least one characteristic comprises determining a location, size, strength, or combinations thereof of the vortex.

21. The computer readable media of Claim 19 wherein determining (30) flow comprises determining (34) vorticity for at least an entire heart cycle for each of the locations;
further comprising:

time-decompositing (36) the vorticity for each of the cardiac locations into Fourier components, including a steady state Fourier component; and

wherein extracting (44) comprises identifying (42), as a function of the steady state Fourier component, the vortex associated with a plurality of the locations.

30 —— Detect Flow with Ultrasound

32 —— Detect Velocity

34 —— Determine Vorticity

36 —— Time-Decomposite Flow Information

37 —— Calculate Steady State

38 —— Calculate First Harmonic Flow

40 —— Generate Image

42 —— Identify Vortex

44 —— Extract Vortex Characteristic

46 —— Output Vortex Information

## Figure 1

14 —— XDCR

12 —— Transmit Beamformer

16 —— Receive Beamformer

10

22 —— Memory

18 —— Processor

20 —— Display

## Figure 5

Figure 2

Figure 3

Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 92612407 P